# EUROPEAN PATENT SPECIFICATION

(11) **EP 4 394 376 B1**
(45) Date of publication and mention of the grant of the patent: **10.06.2026**
(21) Application number: 23220166.5
(22) Date of filing: 22.12.2023
(51) Int. Cl.: G01N 33/00, G01N 15/0227, G01N 15/02, G01N 15/06, G02B 21/26, G03H 1/04

(54) **AN AIR ANALYSIS DEVICE**
LUFTANALYSEVORRICHTUNG
DISPOSITIF D'ANALYSE D'AIR

(30) Priority: 30.12.2022 TR 202221715
(43) Date of publication of application: 03.07.2024
(73) Proprietor: Arçelik Anonim Sirketi, 34445 Istanbul (TR)
(72) Inventor: ALPTEKIN, Ahmet, 34445 Istanbul (TR); DEMIRHAN, Dogan, 34445 Istanbul (TR); SEZER, Sefa, 34445 Istanbul (TR)

(56) References cited:
- WO-A1-2011/049965
- WO-A1-2016/064934
- WO-A1-2021/097225
- US-A1- 2019 293 539
- US-A1- 2021 223 155
- US-A1- 2022 348 861

## Description

The present invention relates to an air analysis device which has a camera positioning mechanism.

In air analysis devices, especially in devices where pollen is detected, the camera should be close to the analyzed cover glass surface in order to receive accurate data. If the distance is not close enough, pollen particles cannot be distinguished from each other properly. In this case, the air analysis device cannot fulfill its main function.

In the state of the art International Patent Application No. WO2018165590, a portable air analysis device is disclosed.

In another state of the art International Patent Application No. WO2013070287, an analysis device is disclosed, wherein the sample is evaluated by means of an image.

US2019293539 discloses an ambient air analysis device wherein sampled air enters through an intake slot, passes a cartridge intake zone where adhesive tape captures particles. A reel motor advances tape for camera inspection of collected particles.

The aim of the present invention is the realization of an air analysis device wherein the cover glass is positioned with respect to the camera.

The air analysis device realized in order to attain the aim of the present invention, explicated in independent claim 1 and the respective claims thereof, comprises a body; at least one cover glass whereon the sample is placed; a camera; a camera holder which is positioned on the body and whereon the camera is fixed; at least one spring which is disposed between the body and the camera holder and which pushes the camera holder upwards in the vertical direction; a holder whereon the cover glass is fixed; a cartridge whereon the holder is placed; a sliding member which is positioned on the body so as to align with the camera and which enables the cartridge thereon to slide and move horizontally; at least one recess which is provided in the side wall of the cartridge; and a protrusion which extends vertically from the camera holder and which is fitted into the recess so as to enable the cartridge to be centered on the camera.

The cartridge comprises a frame; a holder which is placed on the frame and whereon the cover glass is fixed; and a handle which is provided in front of the frame and which enables the user to slide the cartridge back and forth on the sliding member. The cartridge further comprises a slide which is provided on the side wall and which enables the cartridge to slidably move on the body. The particles in the air to be analyzed by the air analysis device adhere to the cover glass, preferably to the gel on the cover glass. The user fixes the cover glass on the frame together with the holder and pushes and slides the cartridge onto the body by means of the handle.

The sliding member comprises a base, side walls in the form of rails on both sides of the base, and a window on the base. When the sliding member is fixed on the body, the window is positioned between the cover glass and the camera. The cartridge is attached on the sliding member, and hence on the body, so as to move back and forth in a horizontal plane as the slide is fitted in the side walls. While the user inserts and removes the cartridge from the air analysis device, the slides on the cartridge move in the side wall.

In the embodiment of the present invention, the air analysis device comprises four springs which are squeezed between the camera holder and the body, and spring housings where the springs are placed in the body. Said springs apply upward force on the vertical axis to the camera holder attached on the body and pushes the camera holder towards the sliding member.

In an embodiment of the present invention, the air analysis device comprises at least one first stopper which is provided on the body and which limits the final position that the camera holder can reach in the body, and at least one first claw which extends downward from the camera holder and which prevents the camera holder from moving further in the vertical axis upon contacting the first stopper. The upward force in the vertical axis applied by the springs to the camera holder is countered as the first claw bears against the first stopper, and the upward movement of the camera holder in the vertical axis on the body is limited.

In another embodiment of the present invention, the air analysis device comprises at least one second claw which extends downwards from the sliding member and which enables the sliding member to be placed and fixed on the body such that the camera and camera holder remain therebelow, and at least one second stopper which is provided on the side wall of the body.

In the embodiment of the present invention, the camera holder is attached on the body so as to be positioned on the springs. Meanwhile, the first claws bear against the first stopper. Then, the sliding member is fixed on the body so as to be positioned above the camera and the camera holder. While the user inserts the cartridge into the air analysis device, the cartridge slides on the sliding member and moves in the horizontal plane by means the slides entering the side wall of the sliding member. During this movement of the cartridge, the rear wall of the cartridge contacts the protrusion, and when the force applied to the protrusion overcomes the force of the springs, the rear wall, together with the protrusion, pushes the camera holder downwards in the vertical plane towards the base of the body. Meanwhile, the springs which support the camera holder are compressed. This movement continues until the protrusion on the camera holder is fitted into the recess on the side wall of the cartridge. When the protrusion reaches the recess, the camera holder starts to move upwards by means of the force of the spring. When the protrusion is completely fitted into the recess, the movement of the cartridge along the sliding member and the upward movement of the camera holder in the vertical plane end. In this case, the cover glass, the window and the camera align with each other, and the camera is brought to the position where the air analysis device can make the most accurate analysis.

By means of the present invention, the cover glass is enabled to be placed correctly in the air analysis device without requiring the user to make any additional intervention to the camera. Moreover, since the distance between the cover glass and the camera is enabled to be constant during all analysis processes, the air analysis device is enabled to make accurate measurements and thus unnecessary use of the cover glass is prevented. Furthermore, the camera is enabled to remain clean while the cover glass is inserted into and removed from the air analysis device.

An air analysis device realized in order to attain the aim of the present invention is illustrated in the attached figures, where:
Figure 1 - is the general view of an air analysis device.
Figure 2 - is the cross-sectional view of the air analysis device.
Figure 3 - the exploded view of the air analysis device.
Figure 4 - is the perspective view of a cartridge.
Figure 5 - is the perspective view of a camera and a camera holder.
Figure 6 - is the perspective view of the body, and the camera and the camera holder placed into the body.
Figure 7 - is the front view of the body, and the camera and the camera holder placed into the body.
Figure 8 - is the cross-sectional view of the body, and the camera and the camera holder placed into the body while the cartridge is placed.
Figure 9 - is the cross-sectional view of the body, and the camera and the camera holder placed into the body, when the cartridge is in place.
Figure 10 - is the sideways view of the cartridge and the camera holder in the initial position.
Figure 11 - is the sideways view of the cartridge and the camera holder after passing the initial position.
Figure 12 - is the sideways view of the cartridge and the camera holder before the reaching the final position.
Figure 13 - is the sideways view of the cartridge and the camera holder in the final position.
Figure 14 - is the sideways view of the cartridge and the camera holder when locked in the final position.

The elements illustrated in the figures are numbered as follows.
1. Air analysis device
2. Body
3. Camera
4. Camera holder
5. Cartridge
6. Frame
7. Handle
8. Recess
9. Cover glass
10. Holder
11. Sliding member
12. Spring
13. Spring housing
14. First stopper
15. First claw
16. Second stopper
17. Second claw
18. Slide
19. Side wall
20. Window
21. Protrusion

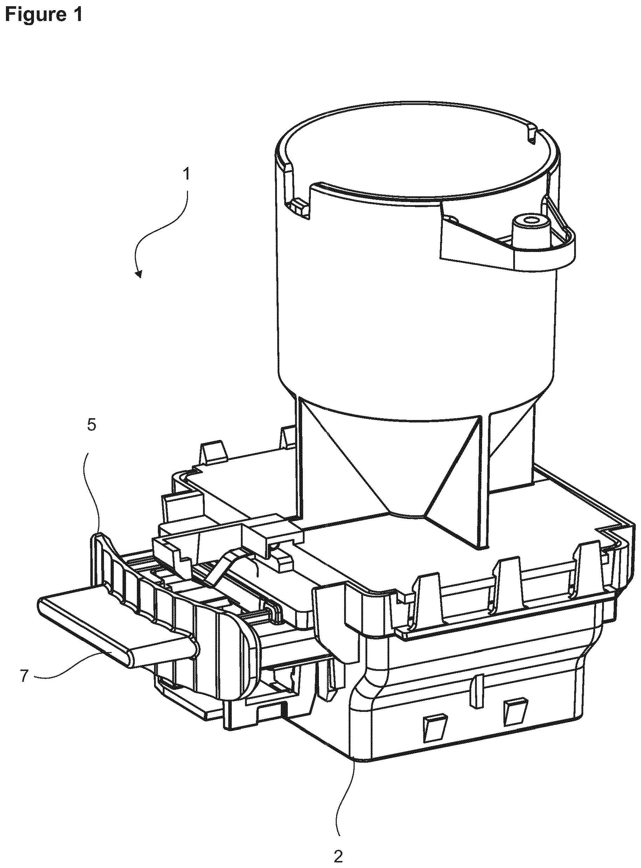

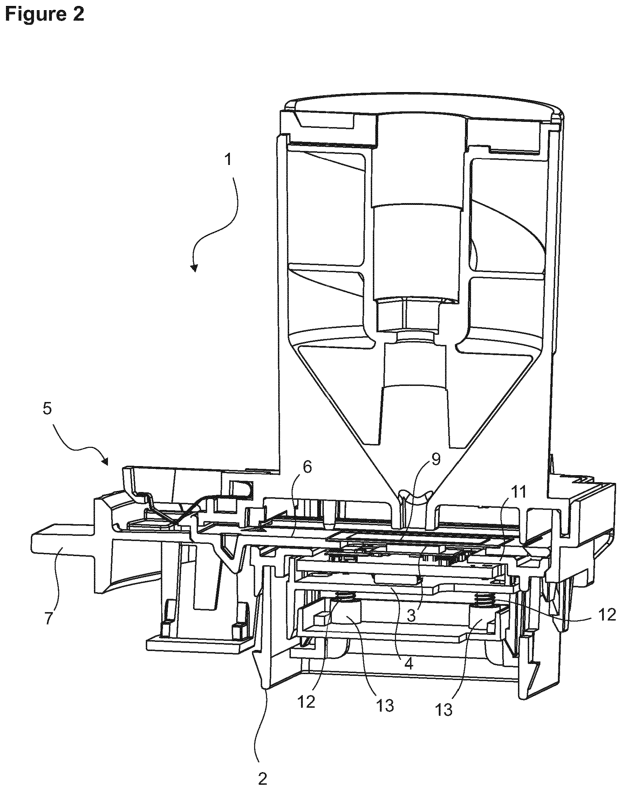

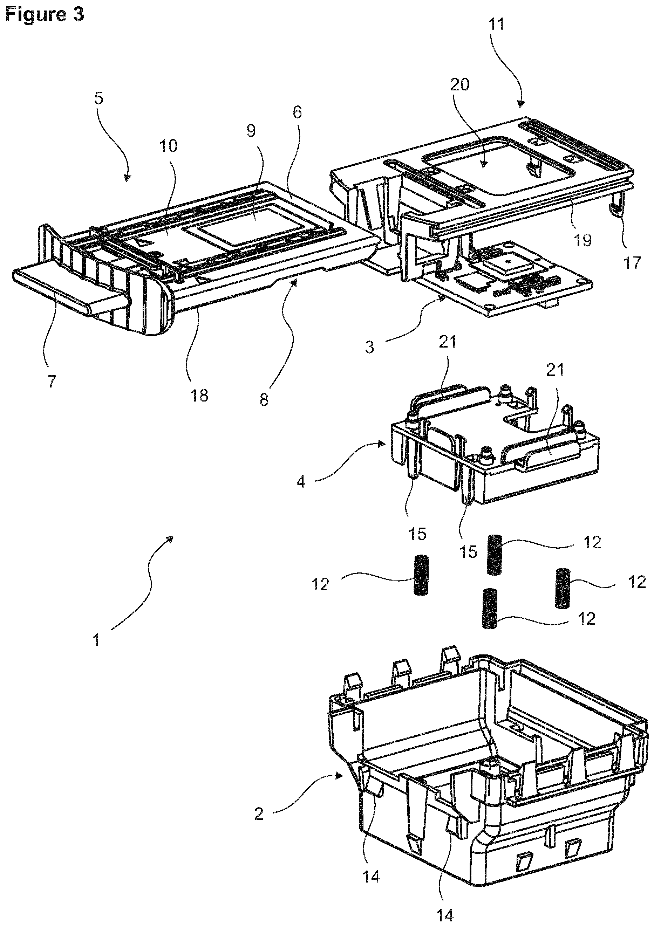

The air analysis device (1) comprises a body (2); at least one cover glass (9) whereon the sample is placed; a camera (3); a camera holder (4) which is positioned on the body (2) and whereon the camera (3) is fixed; at least one spring (12) which is disposed between the body (2) and the camera holder (4) and which pushes the camera holder (4) upwards in the vertical direction; a holder (10) whereon the cover glass (9) is fixed; a cartridge (5) whereon the holder (10) is placed; a sliding member (11) which is positioned on the body (2) so as to align with the camera (3) and which enables the cartridge (5) thereon to slidingly move horizontally; at least one recess (8) which is provided in the side wall of the cartridge (5); and a protrusion (21) which extends perpendicularly from the camera holder (4) to extend vertically and which is fitted into the recess (8) so as to enable the cartridge (5) to be centered on the camera (3) (Figure 1, Figure 2 and Figure 3).

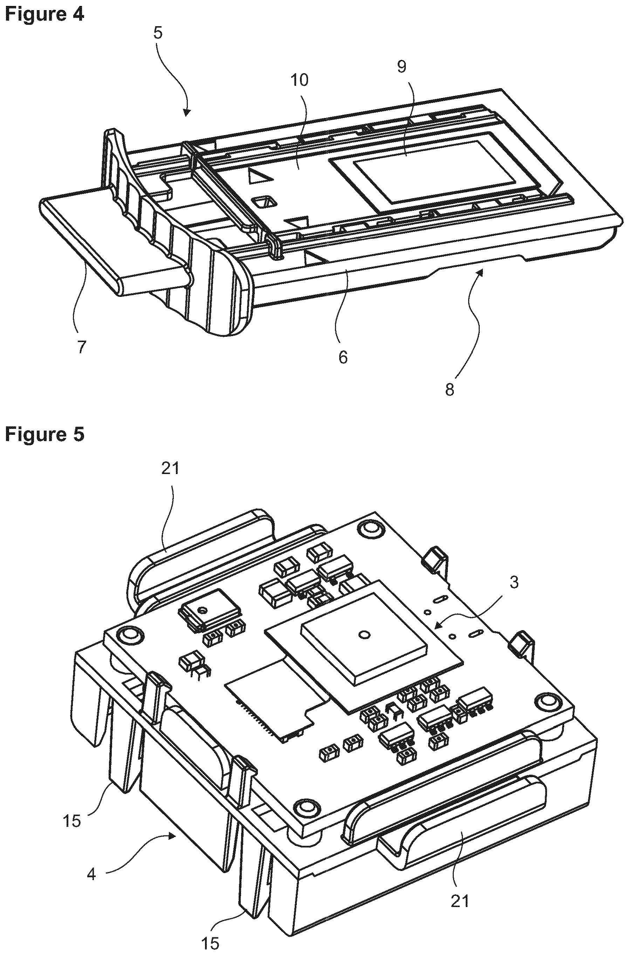

The cartridge (5) comprises a frame (6); a holder (10) which is placed on the frame (6) and whereon the cover glass (9) is fixed; and a handle (7) which is provided in front of the frame (6) and which enables the user to slide the cartridge (5) back and forth on the sliding member (11). The cartridge (5) further comprises a slide (18) which is provided on the side wall and which enables the cartridge to slidably move on the body (2). The particles in the air to be analyzed by the air analysis device (1) adhere to the cover glass (9), preferably to the gel on the cover glass (9). The user fixes the cover glass (9) on the frame (6) together with the holder (10) and pushes and slides the cartridge (5) onto the body (2) by means of the handle (7) (Figure 4).

The sliding member (11) comprises a base, side walls (19) in the form of rails on both sides of the base, and a window (20) on the base. When the sliding member (11) is fixed on the body (2), the window (20) is positioned between the cover glass (9) and the camera (3). The cartridge (5) is attached on the sliding member (11), and hence on the body (2), so as to move back and forth in a horizontal plane as the slide (18) is fitted in the side walls (19). While the user inserts and removes the cartridge (5) from the air analysis device (1), the slides (18) on the cartridge (5) move in the side wall (19).

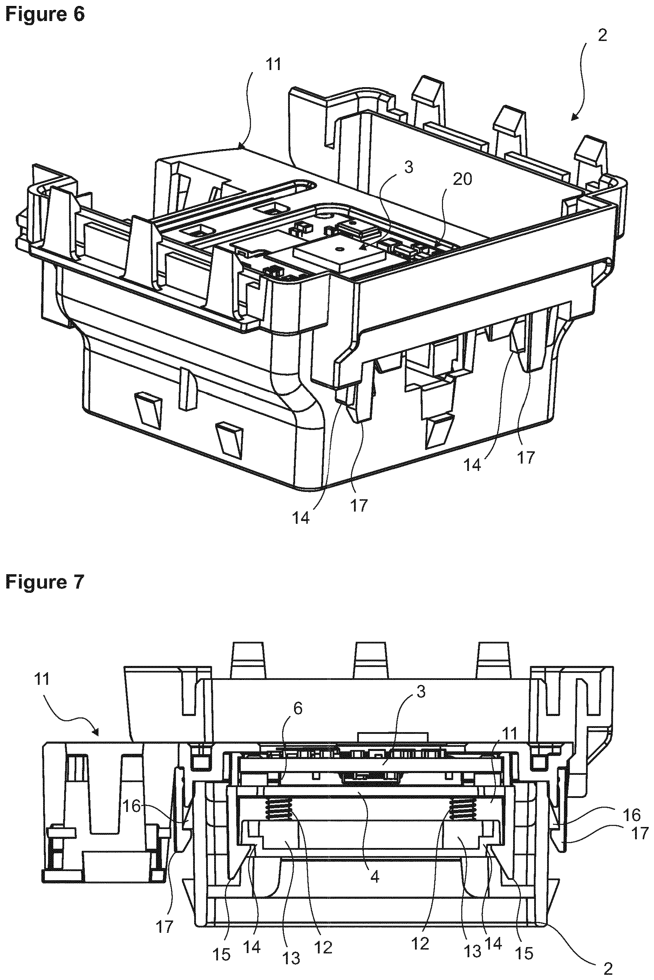

In the embodiment of the present invention, the air analysis device (1) comprises four springs (12) which are squeezed between the camera holder (4) and the body (2), and spring housings (13) where the springs (12) are placed in the body (2). Said springs (12) apply upward force on the vertical axis to the camera holder (4) attached on the body (2) and pushes the camera holder (4) towards the sliding member (11) (Figure 5, Figure 6 and Figure 7).

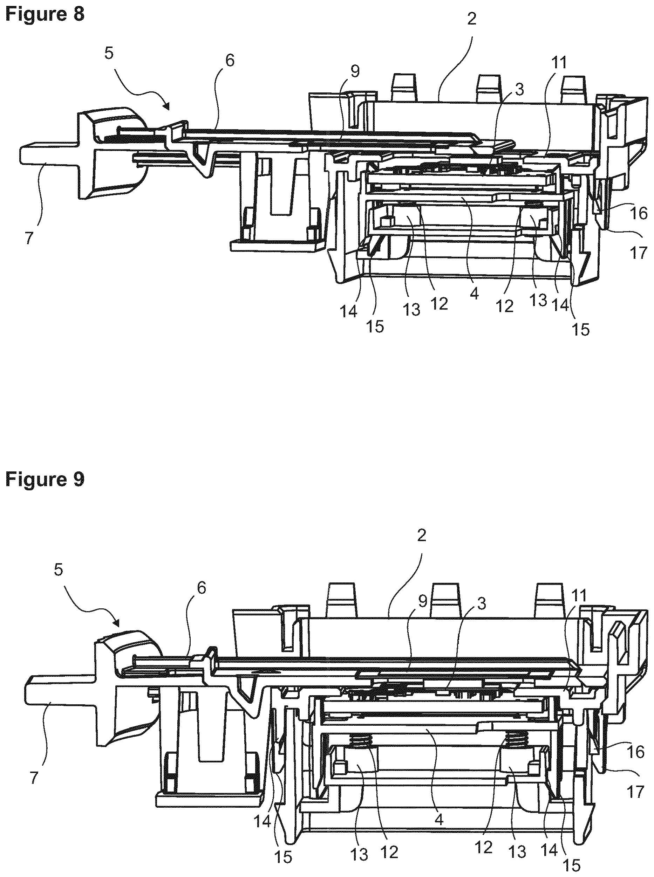

In an embodiment of the present invention, the air analysis device (1) comprises at least one first stopper (14) which is provided on the body (2) and which limits the final position that the camera holder (4) can reach in the body (2), and at least one first claw (15) which extends downward from the camera holder (4) and which prevents the camera holder (4) from moving further in the vertical axis upon contacting the first stopper (14). The upward force in the vertical axis applied by the springs (12) to the camera holder (4) is countered as the first claw (15) bears against the first stopper (14), and the upward movement of the camera holder (4) in the vertical axis on the body (2) is limited (Figure 8 and Figure 9).

In another embodiment of the present invention, the air analysis device (1) comprises at least one second claw (17) which extends downwards from the sliding member (11) and which enables the sliding member (11) to be placed and fixed on the body (2) such that the camera (3) and camera holder (4) remain therebelow, and at least one second stopper (16) which is provided on the side wall of the body (2).

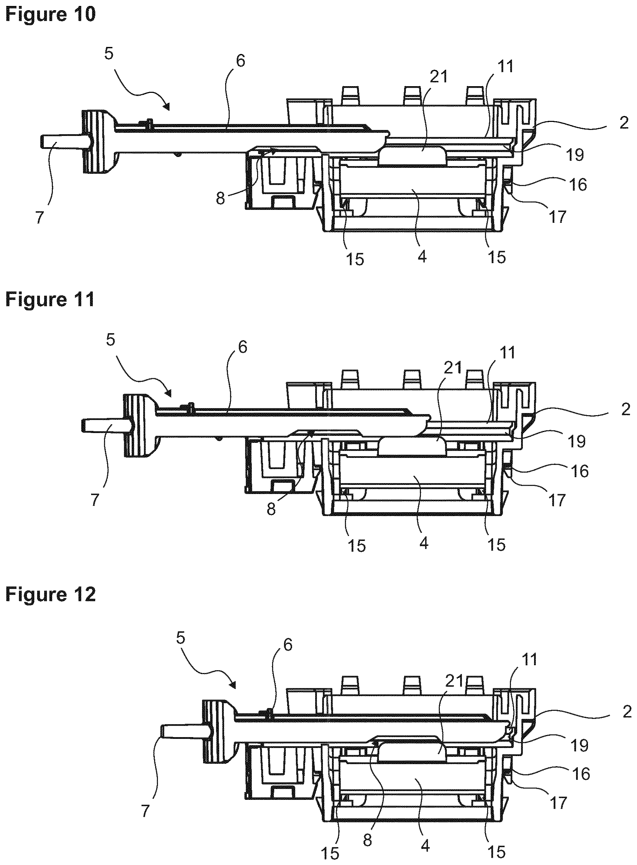

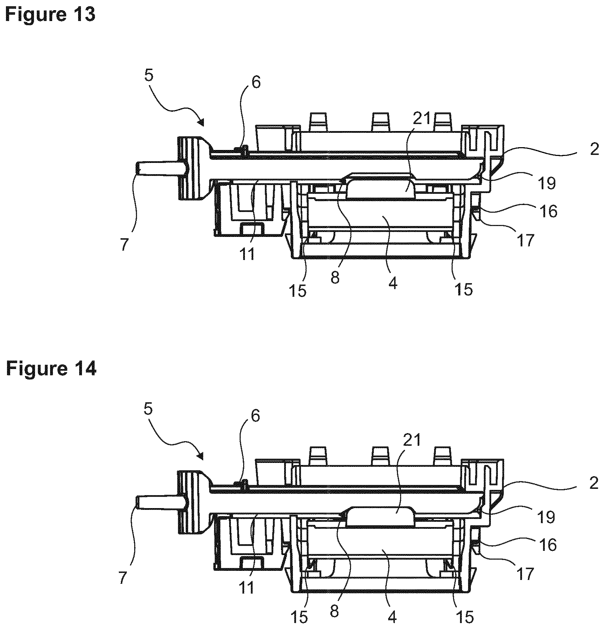

In the embodiment of the present invention, the camera holder (4) is attached on the body (2) so as to be positioned on the springs (12). Meanwhile, the first claws (15) bear against the first stopper (14). Then, the sliding member (11) is fixed on the body (2) so as to be positioned above the camera (3) and the camera holder (4). While the user inserts the cartridge (5) into the air analysis device (1), the cartridge (5) slides on the sliding member (11) and moves in the horizontal plane by means the slides (18) entering the side wall (19) of the sliding member (11) (Figure 10). During this movement of the cartridge (5), the rear wall of the cartridge (5) contacts the protrusion (21), and when the force applied to the protrusion (21) overcomes the force of the springs (12), the rear wall, together with the protrusion (21), pushes the camera holder (4) downwards in the vertical plane towards the base of the body (2) (Figure 11). Meanwhile, the springs (12) which support the camera holder (4) are compressed. This movement continues until the protrusion (21) on the camera holder (4) is fitted into the recess (8) on the side wall of the cartridge (5). When the protrusion (21) reaches the recess (8), the camera holder (4) starts to move upwards by means of the force of the spring (12) (Figure 12 and Figure 13). When the protrusion (21) is completely fitted into the recess (8), the movement of the cartridge (5) along the sliding member (11) and the upward movement of the camera holder (4) in the vertical plane end. In this case, the cover glass (9), the window (20) and the camera (3) align with each other, and the camera (3) is brought to the position where the air analysis device (1) can make the most accurate analysis.

By means of the present invention, the cover glass (9) is enabled to be placed correctly in the air analysis device (1) without requiring the user to make any additional intervention to the camera (3). Moreover, since the distance between the cover glass (9) and the camera (3) is enabled to be constant during all analysis processes, the air analysis device (1) is enabled to make accurate measurements and thus unnecessary use of the cover glass (9) is prevented. Furthermore, the camera (3) is enabled to remain clean while the cover glass (9) is inserted into and removed from the air analysis device (1).

## Claims

1. An air analysis device (1) **comprising** a body (2); at least one cover glass (9) whereon the sample is placed; a camera (3); and a camera holder (4) which is positioned on the body (2) and whereon the camera (3) is fixed, **characterized by** at least one spring (12) which is disposed between the body (2) and the camera holder (4) and which pushes the camera holder (4) upwards in the vertical direction; a holder (10) whereon the cover glass (9) is fixed; a cartridge (5) whereon the holder (10) is placed; a sliding member (11) which is positioned on the body (2) so as to align with the camera (3) and which enables the cartridge (5) thereon to slidingly move horizontally; at least one recess (8) which is provided in the side wall of the cartridge (5); and a protrusion (21) which extends perpendicularly from the camera holder (4) to extend vertically and which is fitted into the recess (8) so as to enable the cartridge (5) to be centered on the camera (3).

2. An air analysis device (1) as in Claim 1, **characterized by** the cartridge (5) comprising a frame (6); a holder (10) which is placed on the frame (6) and whereon the cover glass (9) is fixed; and a handle (7) which is provided in front of the frame (6) and which enables the user to slide the cartridge (5) back and forth on the sliding member (11).

3. An air analysis device (1) as in Claim 2, **characterized by** the cartridge (5) comprising a slide (18) which is provided on the side wall and which enables the cartridge to slidably move on the body (2).

4. An air analysis device (1) as in Claim 1, **characterized by** the sliding member (11) comprising a base, side walls (19) in the form of rails on both sides of the base, and a window (20) on the base.

5. An air analysis device (1) as in Claim 4, **characterized by** the window (20) which is positioned between the cover glass (9) and the camera (3) when the sliding member (11) is fixed on the body (2).

6. An air analysis device (1) as in Claim 1, **characterized by** four springs (12) which are squeezed between the camera holder (4) and the body (2), and spring housings (13) where the springs (12) are placed in the body (2).

7. An air analysis device (1) as in Claim 6, **characterized by** the spring (12) which apply upward force on the vertical axis to the camera holder (4) attached on the body (2) and pushes the camera holder (4) towards the sliding member (11).

8. An air analysis device (1) as in Claim 1, **characterized by** at least one first stopper (14) which is provided on the body (2) and which limits the final position that the camera holder (4) can reach in the body (2), and at least one first claw (15) which extends downward from the camera holder (4) and which prevents the camera holder (4) from moving further in the vertical axis upon contacting the first stopper (14).

9. An air analysis device (1) as in Claim 1, **characterized by** at least one second claw (17) which extends downwards from the sliding member (11) and which enables the sliding member (11) to be placed and fixed on the body (2) such that the camera (3) and camera holder (4) remain therebelow, and at least one second stopper (16) which is provided on the side wall of the body (2).

## Patentansprüche

1. Ein Luftanalysegerät (1), umfasst einen Gehäuse (2); mindestens ein Deckglas (9), auf das die Probe aufgelegt wird; eine Kamera (3); und einen Kamerahalter (4), der auf dem Gehäuse (2) positioniert ist und an dem die Kamera (3) befestigt ist, **gekennzeichnet ist es durch** mindestens eine Feder (12), die zwischen dem Gehäuse (2) und dem Kamerahalter (4) angeordnet ist und den Kamerahalter (4) in vertikaler Richtung nach oben drückt; einen Halter (10), an dem das Deckglas (9) befestigt ist; eine Kassette (5), auf der der Halter (10) angeordnet ist; ein Gleitelement (11), das auf dem Gehäuse (2) so positioniert ist, dass es mit der Kamera (3) fluchtet, und das es ermöglicht, dass sich die darauf befindliche Kassette (5) horizontal verschieben lässt; mindestens eine Aussparung (8), die in der Seitenwand der Kassette (5) vorgesehen ist; und einen Vorsprung (21), der sich senkrecht vom Kamerahalter (4) aus erstreckt, um sich vertikal zu erstrecken, und der in die Aussparung (8) eingepasst ist, um zu ermöglichen, dass die Kassette (5) auf der Kamera (3) zentriert wird.

2. Ein Luftanalysegerät (1), wie in Anspruch 1 aufgeführt, **ist dadurch gekennzeichnet, dass** die Kassette (5) einen Rahmen (6), einen Halter (10), der auf dem Rahmen (6) angebracht ist und an dem das Deckglas (9) befestigt ist, sowie einen Griff (7) umfasst, der vor dem Rahmen (6) vorgesehen ist und es dem Benutzer ermöglicht, die Kassette (5) auf dem Gleitelement (11) hin und her zu schieben.

3. Ein Luftanalysegerät (1), wie in Anspruch 2 aufgeführt, **ist dadurch gekennzeichnet, dass** die Kassette (5) einen an der Seitenwand angeordneten Schieber (18) aufweist, der es ermöglicht, die Kassette auf dem Gehäuse (2) verschiebbar zu bewegen.

4. Ein Luftanalysegerät (1), wie in Anspruch 1 aufgeführt, **ist dadurch gekennzeichnet, dass** das Schiebeelement (11) eine Basis, Seitenwände (19) in Form von Schienen an beiden Seiten der Basis und ein Fenster (20) an der Basis umfasst.

5. Ein Luftanalysegerät (1), wie in Anspruch 4 aufgeführt, **ist dadurch gekennzeichnet, dass** sich das Fenster (20) zwischen dem Deckglas (9) und der Kamera (3) befindet, wenn das Schiebeelement (11) am Gehäuse (2) befestigt ist.

6. Ein Luftanalysegerät (1), wie in Anspruch 1 aufgeführt, **ist dadurch gekennzeichnet, dass** vier Federn (12), die zwischen dem Kamerahalter (4) und dem Gehäuse (2) eingeklemmt sind, sowie Federgehäuse (13), in denen die Federn (12) im Gehäuse (2) untergebracht sind.

7. Ein Luftanalysegerät (1), wie in Anspruch 6 aufgeführt, **ist dadurch gekennzeichnet, dass** die Feder (12) eine nach oben gerichtete Kraft auf die vertikale Achse des am Gehäuse (2) befestigten Kamerahalters (4) ausübt und den Kamerahalter (4) in Richtung des Gleitelements (11) drückt.

8. Ein Luftanalysegerät (1), wie in Anspruch 1 aufgeführt, **ist dadurch gekennzeichnet, dass** mindestens ein erster Anschlag (14), der am Gehäuse (2) vorgesehen ist und die Endposition begrenzt, die der Kamerahalter (4) im Gehäuse (2) erreichen kann, sowie mindestens eine erste Klaue (15), die sich vom Kamerahalter (4) nach unten erstreckt und verhindert, dass sich der Kamerahalter (4) bei Kontakt mit dem ersten Anschlag (14) weiter in vertikaler Richtung bewegt.

9. Ein Luftanalysegerät (1), wie in Anspruch 1 aufgeführt, **ist dadurch gekennzeichnet, dass** mindestens eine zweite Klaue (17), die sich vom Gleitelement (11) nach unten erstreckt und die es ermöglicht, das Gleitelement (11) so auf dem Gehäuse (2) anzubringen und zu befestigen, dass die Kamera (3) und der Kamerahalter (4) darunter verbleiben, sowie mindestens ein zweiter Anschlag (16), der an der Seitenwand des Gehäuses (2) vorgesehen ist.

## Revendications

1. Un dispositif d'analyse de l'air (1) **coomprenant** un corps (2) ; au moins une lame de verre de couverture (9) sur laquelle l'échantillon est placé ; une caméra (3) ; et un support de caméra (4) qui est positionné sur le corps (2) et sur lequel la caméra (3) est fixée, **caractérisé par** au moins un ressort (12) qui est disposé entre le corps (2) et le support de caméra (4) et qui pousse le support de caméra (4) vers le haut dans la direction verticale ; un support (10) sur lequel la lame de verre de couverture (9) est fixée ; une cartouche (5) sur laquelle le support (10) est placé ; un élément coulissant (11) qui est positionné sur le corps (2) de manière à s'aligner avec la caméra (3) et qui permet à la cartouche (5) qui s'y trouve de se déplacer horizontalement par coulissement ; au moins un évidement (8) qui est prévu dans la paroi latérale de la cartouche (5) ; et une saillie (21) qui s'étend perpendiculairement depuis le support de caméra (4) de manière à s'étendre verticalement et qui est engagée dans l'évidement (8) afin de permettre le centrage de la cartouche (5) par rapport à la caméra (3).

2. Dispositif d'analyse de l'air (1) selon la Revendication 1, **caractérisé par** la cartouche (5) comprenant un cadre (6) ; un support (10) qui est placé sur le cadre (6) et sur lequel la lame de verre de couverture (9) est fixée ; et une poignée (7) qui est disposée à l'avant du cadre (6) et qui permet à l'utilisateur de faire coulisser la cartouche (5) d'avant en arrière sur l'élément coulissant (11).

3. Dispositif d'analyse de l'air (1) selon la Revendication 2, **caractérisé par** la cartouche (5) comprenant une glissière (18) qui est prévue sur la paroi latérale et qui permet à la cartouche de se déplacer de manière coulissante sur le corps (2).

4. Dispositif d'analyse de l'air (1) selon la Revendication 1, **caractérisé par** l'élément coulissant (11) comprenant une base, des parois latérales (19) sous forme de rails de part et d'autre de la base, et une fenêtre (20) sur la base.

5. Dispositif d'analyse de l'air (1) selon la Revendication 4, **caractérisé par** la fenêtre (20) qui est positionnée entre la lame de verre de couverture (9) et la caméra (3) lorsque l'élément coulissant (11) est fixé sur le corps (2).

6. Dispositif d'analyse de l'air (1) selon la Revendication 1, **caractérisé par** quatre ressorts (12) qui sont comprimés entre le support de caméra (4) et le corps (2), et des logements de ressorts (13) dans lesquels les ressorts (12) sont placés dans le corps (2).

7. Dispositif d'analyse de l'air (1) selon la Revendication 6, **caractérisé par** les ressorts (12) qui appliquent une force vers le haut sur l'axe vertical au support de caméra (4) fixé sur le corps (2) et qui poussent le support de caméra (4) vers l'élément coulissant (11).

8. Dispositif d'analyse de l'air (1) selon la Revendication 1, **caractérisé par** au moins une première butée (14) qui est prévue sur le corps (2) et qui limite la position finale que peut atteindre le support de caméra (4) dans le corps (2), et au moins un premier ergot (15) qui s'étend vers le bas depuis le support de caméra (4) et qui empêche le support de caméra (4) de se déplacer davantage selon l'axe vertical lors du contact avec la première butée (14).

9. Dispositif d'analyse de l'air (1) selon la revendication 1, **caractérisé par** au moins un second ergot (17) qui s'étend vers le bas depuis l'élément coulissant (11) et qui permet de placer et de fixer l'élément coulissant (11) sur le corps (2) de sorte que la caméra (3) et le support de caméra (4) se trouvent en dessous, et au moins une seconde butée (16) qui est prévue sur la paroi latérale du corps (2).
